# FASCICULE DE BREVET EUROPEEN

(11) **EP 2 549 911 B1**
(45) Date de publication et mention de la délivrance du brevet: **13.11.2019**
(21) Numéro de dépôt: 11710712.8
(22) Date de dépôt: 24.03.2011
(51) Int. Cl.: A61B 1/00, A61M 25/01, A61B 1/005

(54) **PROCEDE DE FABRICATION D' UNE STRUCTURE ALLONGEE SOUPLE A EXTREMITE ORIENTABLE**
VERFAHREN ZUR HERSTELLUNG EINER BIEGSAMEN LÄNGLICHEN STRUKTUR MIT AUSRICHTBAREM ENDE
PROCESS FOR MANUFACTURING A FLEXIBLE ELONGATE STRUCTURE HAVING AN ORIENTABLE END

(30) Priorité: 24.03.2010 FR 1052119
(43) Date de publication de la demande: 30.01.2013
(73) Titulaire: Sorbonne Université, 75006 Paris (FR); Centre National de la Recherche Scientifique (C.N.R.S.), 75016 Paris (FR)
(72) Inventeur: SZEWCZYK, Jérôme, F-95510 Vienne en Arthies (FR)
(74) Mandataire: Parzy, Benjamin Alain
(86) Numéro de dépôt international: PCT/EP2011/001472
(87) Numéro de publication internationale: WO 2011/116961

(56) Documents cités:
- WO-A1-95/06494
- US-A- 4 944 727

## Description

La présente invention concerne un procédé de fabrication d'une structure allongée souple à extrémité orientable.

L'invention trouvera avantageusement application dans le domaine médical et plus particulièrement pour la réalisation d'un endoscope ou encore d'un cathéter.

Toutefois bien que particulièrement prévu pour des applications médicales, l'invention pourra également être utilisée dans d'autres domaines techniques variés faisant appel à des structures allongées à extrémités orientables tels que le contrôle non destructif de canalisations.

### ARRIERE PLAN DE L'INVENTION

De manière connue, les chirurgiens privilégient les techniques peu invasives, mettant en jeu des voies d'accès étroites pour traiter les pathologies vasculaires. Ces techniques permettent en effet de limiter le recours à la chirurgie ouverte et par conséquent sont moins lourdes pour le patient. A cette fin on utilise souvent des cathéters ou endoscopes possédant à leur extrémité un corps allongé orientable afin de faciliter l'insertion et la progression du corps allongé à l'intérieur du corps humain.

La structure du corps allongé comprend au moins un organe d'actionnement permettant de courber l'extrémité afin de pouvoir négocier les virages et se déplacer dans des parties non rectilignes du corps humain ou encore de visualiser ou de traiter des parties du corps qui ne sont pas disposées dans l'axe principal du corps allongé.

Différents types d'organes d'actionnement ont été proposés, certaines utilisant des fils en alliage à mémoire de forme (appelés ci-dessous FAMFs).

Les FAMFs s'étendent le long du corps allongé, leurs extrémités étant ancrées sur celui-ci. Les FAMFs sont associés à des moyens de leur chauffage, par exemple par effet Joule, provoquant leur contraction, en vue de provoquer une flexion du corps allongé. A cet effet, des fils d'alimentation électrique sont connectés aux FAMFs.

Cependant, ces structures sont difficiles à fabriquer. En effet, le FAMF doit d'une part être fixé au niveau de ses extrémités, sur le corps longitudinal, et d'autre part être relié au fil d'alimentation.

Différents mode d'assujettissement des FAMFs sur le corps, par collage ou encore par laser ont été proposés, mais ils ne sont guère satisfaisants car ils ne permettent pas de garantir à la fois un positionnement précis du fil sur le corps longitudinal et une bonne tenue dans le temps, notamment compte tenu des fortes variations de température au niveau du FAMF, et des forces de traction élevées subies par le FAMF.

Il est important de souligner que le positionnement du fil est d'autant plus important lorsque le corps longitudinal supporte plusieurs FAMFs, puisqu'il faut en effet empêcher tout contact entre ces fils afin d'éviter un court circuit.

Enfin, il faut également noter que la surépaisseur due aux FAMFs et aux moyens de liaison des FAMFs au corps doit être minimale pour que l'ensemble présente un encombrement faible nécessaire au passage de la structure dans des conduits de petits diamètres.

### OBJET DE L'INVENTION

La présente invention vise à proposer un perfectionnement de ces structures longitudinales orientables comportant des actionneurs à FAMFs.

### RESUME DE L'INVENTION

A cet effet l'invention concerne un procédé de fabrication d'une structure allongée à extrémité orientable comportant un corps longitudinal souple associé à au moins un organe d'actionnement comportant au moins un fil en alliage à mémoire de forme ou FAMF s'étendant le long du corps en étant ancré sur celui-ci à ses extrémités et associé à des moyens de son chauffage commandé.

Selon l'invention, le procédé comprend les étapes suivantes :
- insertion du FAMF dans des tubes, dont au moins deux tubes d'extrémité ;
- sertissage d'au moins les tubes d'extrémités sur des extrémités du FAMF;
- solidarisation des tubes au corps de sorte que le FAMF s'étende le long du corps.

Ainsi, l'utilisation de tubes sertis facilite la manipulation du FAMF lors de son positionnement le long du corps, sans risque de détérioration du FAMF lors de la pose.

Cette technique évite tout soudage ou collage du FAMF. Les sertissages déforment la surface extérieure des tubes, ce qui permet, notamment si les tubes sont ligaturés au corps, une meilleure tenue de la liaison entre les tubes et le corps, au regard des efforts de traction provoqués par la contraction du FAMF.

En outre, le tube peut être choisi électriquement conducteur et ainsi être relié au fil d'alimentation électrique, facilitant ainsi la connexion électrique entre le fil d'alimentation électrique et le FAMF.

Selon un autre aspect de l'invention, on prévoit, entre les tubes d'extrémité sertis aux extrémités du FAMF, des tubes intermédiaires également solidarisés au corps, pouvant être ou non sertis sur le FAMF.

Cette caractéristique permet l'obtention d'une courbure accrue de la portion du corps longitudinal s'étendent entre les tubes d'extrémité et par conséquent facilite l'orientation et le déplacement de la structure longitudinale par le praticien.

### BREVE DESCRIPTION DES DESSINS

La présente invention sera mieux comprise à la lecture d'un exemple détaillé de réalisation en référence aux dessins annexés, fournis à titre d'exemple non limitatif, parmi lesquels :
- la figure 1 est une vue en perspective d'une structure allongée à extrémité orientable selon un mode particulier de réalisation de l'invention ;
- les figures 2a à 2g sont des vues en coupe longitudinale partielle de la structure allongée à extrémité orientable de la figure 1, lors des différentes étapes de sa réalisation ;
- la figure 3 est une vue en coupe longitudinale partielle d'une structure allongée selon un deuxième mode de réalisation de l'invention ;
- les figures 4 et 5 sont des vues en coupe longitudinale partielle d'une variante de réalisation d'une structure longitudinale selon une position au repos et une position fléchie.

### DESCRIPTION DETAILLEE DE L'INVENTION

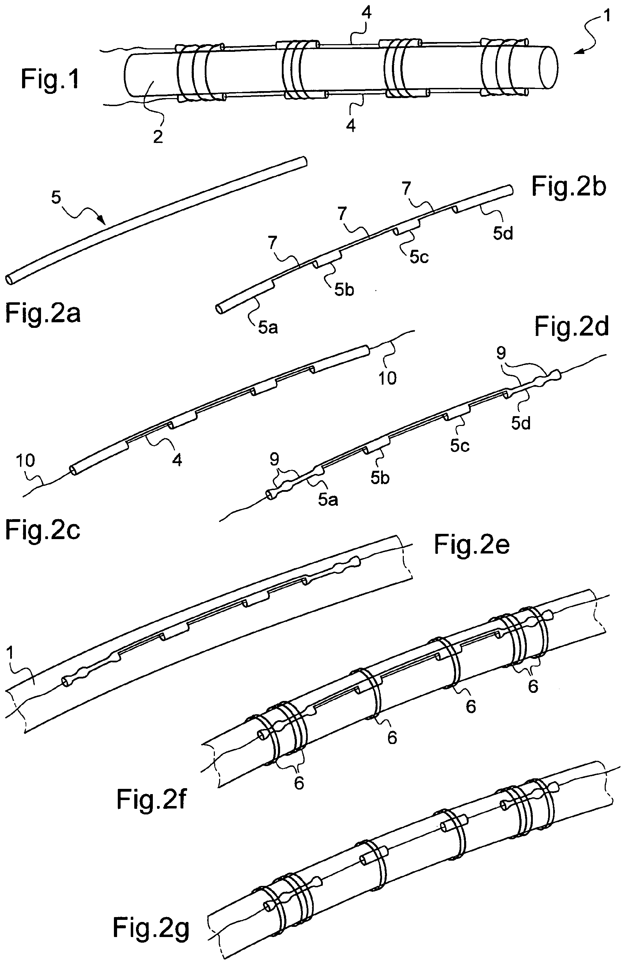

La figure 1 illustre une structure longitudinale à extrémité orientable 1 comportant un corps longitudinal souple 2 associé à un organe d'actionnement 3 qui comporte un FAMF 4 s'étendant le long du corps 2. Le FAMF s'étend à l'intérieur d'un certain nombre de tubes 5a,5b,5c,5d qui sont solidarisés au corps 2 par des ligaments 7. Ici, tous les tubes sont sertis sur le FAMF 4. Les tubes sont ici électriquement conducteurs, et les tubes d'extrémité 5a et 5d sont connectés à des fils d'alimentation électrique permettant de chauffer le FAMF 4 par effet Joule, ce qui provoque sa contraction et donc la flexion de la partie du corps 2 le long de laquelle le FAMF 4 s'étend.

Selon un premier mode de fabrication dont les étapes sont illustrées aux figures 2a à 2g, on part d'une gaine 5 en matériau conducteur rigide que l'on découpe comme illustré à la figure 2b pour laisser subsister des tronçons tubulaires 5a à 5d reliés entre eux par des ligaments 7. Puis, comme cela est visible à la figure 2c, on met en place le FAMF 4 à l'intérieur des tubes 5 de sorte que celui-ci s'étende dans tous les tubes, puis on met en place les fils d'alimentation électrique 10 en engageant leurs extrémités dans les tubes d'extrémité 5a et 5d respectivement.

Puis, comme visible à la figure 2d, on sertit les tubes d'extrémité 5a,5d sur les fils 4,10, ménageant ainsi sur ceux-ci des successions de creux 9 et de reliefs. Puis, comme visible à la figure 2e, on rapporte l'ensemble ainsi constitué contre le corps 2.

Comme illustré à la figure 2f, on solidarise l'ensemble au corps 2 au moyen de ligatures 6 qui s'étendent pour enserrer les tubes 5a, 5b, 5c, 5d. On remarquera que pour les tubes d'extrémité 5a,5d, les ligatures 6 s'étendent dans les creux 9 de sertissage, réalisant un arrêt axial desdits tubes sur le corps 2.

Enfin, comme illustré à la figure 2g, les ligaments 7 sont éliminés, ne laissant subsister de la gaine initiale que les tubes 5a à 5d. Ainsi, on s'assure que les tubes sont parfaitement alignés entre eux lors de leur solidarisation au corps longitudinal souple 2.

Dans ce mode de réalisation, les tubes d'extrémité 5a et 5d sont sertis sur les extrémités du FAMF 4 et forment ainsi un ancrage de ce dernier sur le corps 2. Ils assurent également une connexion électrique entre le FAMF 4 et les fils d'alimentation électrique 10. Tandis que les tubes intermédiaires 5b et 5c forment des paliers de guidage du FAMF 4 lors de ses contractions et dilatations commandées.

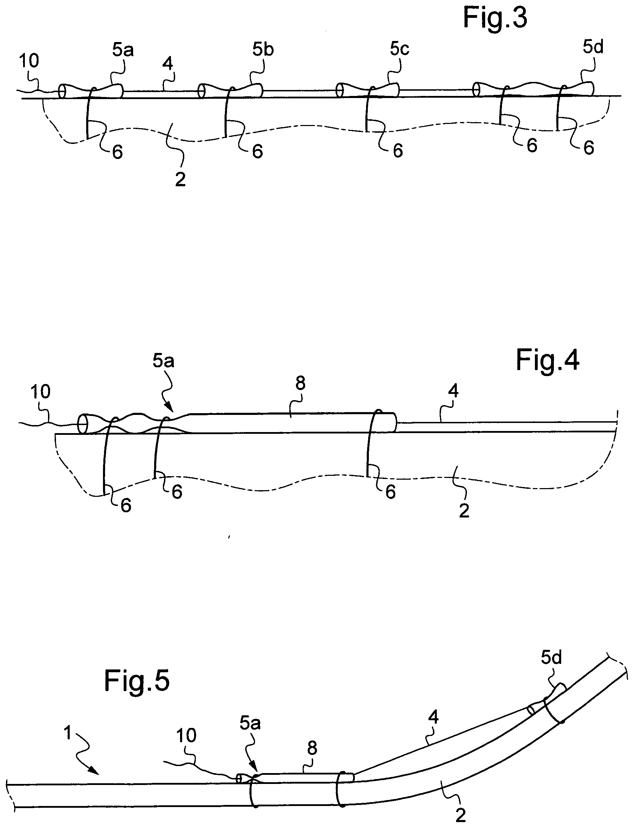

Ce mode de réalisation est susceptible de nombreuses variantes dont certaines sont illustrées sur les figures 3 et 4.

Comme illustré à la figure 3, les ligatures 6 peuvent enserrer plusieurs tubes associés chacun à FAMF 4 distinct. En outre, les tubes intermédiaires peuvent eux-mêmes être sertis sur le FAMF 4.

Comme illustré à la figure 4, on peut laisser subsister sur la gaine une partie tubulaire 8 prolongeant l'un des tubes afin de raidir localement le corps 2. Comme illustré ici, on a laissé subsister une partie tubulaire 8 prolongeant le tube 5a, et dont l'extrémité a été ligaturée au corps 2. Ainsi, et comme cela est visible à la figure 5, le corps 2 ne peut pas, lors de la contraction du FAMF 4, fléchir au niveau de la partie tubulaire 8 mais seulement dans les portions le long desquelles le FAMF est laissé dégagé de tout guidage. Ainsi, le prolongement par une partie tubulaire 8 contribue à diminuer le rayon de courbure du corps lors de sa flexion car celle-ci est concentrée au niveau des portions précitées, pour une même longueur de FAMF.

Bien sûr, les tubes d'extrémité 5a, 5d pourront être prolongés vers l'intérieur, c'est-à-dire en direction des autres tubes comme illustré ici, ou vers l'extérieur. De même, on pourra prolonger les tubes intermédiaires.

De préférence, la longueur du prolongement 8 est inférieure à cinq fois le diamètre du corps longitudinal 2, de sorte que la souplesse globale de ce dernier reste suffisante pour ne pas entraver la progression du corps 2.

L'invention n'est pas limitée à ce qui vient d'être décrit, mais englobe au contraire toutes variante entrant dans le cadre défini par les revendications.

En particulier, bien que l'on ait ici indiqué que l'on solidarisait les tubes au corps par des ligatures, on pourra utiliser d'autres modes de solidarisation, comme un collage.

Bien que l'on ait indiqué que les tubes étaient en matériau électriquement conducteur, ceci n'est pas impératif, bien que cela facilite la connexion entre le FAMF et les fils d'alimentation électrique. Si le diamètre des tubes est suffisant pour superposer à l'intérieur des tubes d'extrémité le FAMF et le fil d'alimentation correspondant, un contact électrique s'instaure alors entre les deux par la pression induite par le sertissage.

Bien que l'on ait indiqué ici que les tubes étaient fabriqués par découpage d'une même gaine, ce qui facilite énormément la manipulation du FAMF et son positionnement sur le corps de la structure, on pourra à la place utiliser plusieurs tubes dans lesquels on enfile un même FAMF, dont au moins deux tubes d'extrémité.

## Revendications

1. Procédé de fabrication d'une structure allongée à extrémité orientable comportant un corps longitudinal (2) souple associé à au moins un organe d'actionnement comportant au moins un fil en alliage à mémoire de forme ou FAMF (4) s'étendant le long du corps (2) en étant ancré sur celui-ci à ses extrémités, le FAMF étant associé à des moyens de son chauffage commandé, **caractérisé en ce que** le procédé comprend les étapes suivantes :
- insertion du FAMF dans des tubes (5a...5d), dont au moins deux tubes d'extrémité (5a, 5d)
- sertissage d'au moins les tubes d'extrémités sur des extrémités du FAMF ;
- solidarisation des tubes au corps (2) de sorte que le fil s'étende le long du corps (2).

2. Procédé selon la revendication 1, dans lequel les tubes (5a, 5b, 5c, 5d) sont tous venus de découpage d'une même gaine (5).

3. Procédé selon la revendication 2, dans lequel la gaine (5) est découpée pour laisser subsister les tubes liés entre eux par des ligaments (7), les tubes ainsi liés étant alors solidarisés au corps avant que les ligaments ne soient éliminés.

4. Procédé selon la revendication 1, dans lequel on insère dans les tubes d'extrémités (5a, 5d) des extrémités de fil d'alimentation électrique (10).

5. Procédé selon la revendication 1, dans lequel on lie les tubes au corps au moyen de ligatures (6) .

6. Procédé selon la revendication 5, dans lequel, pour les tubes sertis, les ligatures s'étendent dans des creux (9) laissés par le sertissage desdits tubes.

7. Procédé selon la revendication 1, dans lequel l'un au moins des tubes est muni d'un prolongement (8) au-delà d'une partie sertie.

## Patentansprüche

1. Verfahren zum Herstellen einer länglichen Struktur mit ausrichtbarem Ende, die einen biegsamen Längskörper (2) umfasst, der mit mindestens einem Betätigungselement verbunden ist, das mindestens einen Formgedächtnislegierungsdraht oder FGL-Draht (4) umfasst, der sich entlang des Körpers (2) erstreckt und dabei an seinen Enden an dem Körper verankert ist, wobei der FGL-Draht mit Mitteln für seine gesteuerte Erwärmung verbunden ist, **dadurch gekennzeichnet, dass** das Verfahren die folgenden Schritte umfasst:
- Einfügen des FGL-Drahtes in Rohre (5a...5d), darunter mindestens zwei Endrohre (5a, 5d);
- Crimpen mindestens der Endrohre auf Enden des FGL-Drahtes;
- Verbinden der Rohre mit dem Körper (2) derart, dass sich der Draht entlang des Körpers (2) erstreckt.

2. Verfahren nach Anspruch 1, bei dem die Rohre (5a, 5b, 5c, 5d) alle durch Schneiden von einem selben Schlauch (5) erhalten werden.

3. Verfahren nach Anspruch 2, bei dem der Schlauch (5) so geschnitten wird, dass die Rohre miteinander über Bänder (7) verbunden bleiben, wobei die so verbundenen Rohre dann mit dem Körper verbunden werden, ehe die Bänder entfernt werden.

4. Verfahren nach Anspruch 1, bei dem man in die Endrohre (5a, 5d) Enden eines Stromzuführungsdrahtes (10) einfügt.

5. Verfahren nach Anspruch 1, bei dem man die Rohre mit dem Körper über Wickeldrähte (6) verbindet.

6. Verfahren nach Anspruch 5, bei dem sich die Wickeldrähte im Falle der gecrimpten Rohre in Vertiefungen (9) erstrecken, die durch das Crimpen der genannten Rohre hinterlassen wurden.

7. Verfahren nach Anspruch 1, bei dem mindestens eines der Rohre mit einer Verlängerung (8) jenseits eines gecrimpten Abschnitts versehen ist.

## Claims

1. A method of fabricating an elongate structure having a steerable end, the structure comprising a flexible longitudinal body (2) associated with at least one actuator member comprising at least one shape memory alloy wire or "SMAW" (4) extending along the body (2) and anchored thereto at its ends, the SMAW being associated with means for heating it in controlled manner, wherein the method comprises the following steps:
• inserting the SMAW in tubes (5a, ..., 5d) including at least two end tubes (5a, 5d);
• crimping at least the end tubes onto the ends of the SMAW; and
• securing the tubes to the body (2) in such a manner that the wire extends along the body (2).

2. A method according to claim 1, wherein the tubes (5a, ..., 5d) are all obtained by being cut out from a common sheath (5).

3. A method according to claim 2, wherein the sheath (5) is cut so as to leave the tubes connected together by ligaments (7), the tubes as connected together in this way then being secured to the body before the ligaments are eliminated.

4. A method according to claim 1, wherein the ends of electrical power supply wires (10) are inserted in the end tubes (5a, 5d).

5. A method according to claim 1, wherein the tubes are connected to the body by means of ligatures (6).

6. A method according to claim 5, wherein, for the crimped tubes, the ligatures extend in indentations (9) left by the crimping of said tubes.

7. A method according to claim 1, wherein at least one of the tubes is provided with an extension (8) beyond a crimped portion.
